# EUROPÄISCHE PATENTSCHRIFT

(11) **EP 0 790 231 B1**
(45) Veröffentlichungstag und Bekanntmachung des Hinweises auf die Patenterteilung: **04.04.2001**
(21) Anmeldenummer: 97101511.0
(22) Anmeldetag: 31.01.1997
(51) Int. Cl.: C07C 68/08, C07C 68/00, C07C 69/96

(54) **Verfahren zur extraktiven Abtrennung von Diarylcarbonaten und den zugrundeliegenden aromatischen Hydroxyverbindungen aus Reaktionslösungen**
Process for the extractive separation of diaryl carbonates and corresponding hydroxyaromatic compounds from reactive solutions
Procédé pour la séparation extractive de carbonates de diaryle et de composés hydroxylés aromatiques correspondants de solutions réactionnels

(30) Priorität: 13.02.1996 DE 19605167
(43) Veröffentlichungstag der Anmeldung: 20.08.1997
(73) Patentinhaber: BAYER AG, 51368 Leverkusen (DE)
(72) Erfinder: Buysch, Hans-Josef, Dr., 47809 Krefeld (DE); Hesse, Carsten, Dr., 47800 Krefeld (DE); Rechner, Johann, Dr., 47906 Kempen (DE)

(56) Entgegenhaltungen:
- US-A- 5 328 615
- US-A- 5 489 703

## Beschreibung

Die vorliegende Erfindung betrifft die Extraktion von Diarylcarbonaten und den zugrundeliegenden aromatischen Hydroxyverbindungen aus Reaktionslösungen der oxidativen Carbonylierung aromatischer Hydroxyverbindungen, wozu man nach der Reaktion die Reaktionslösung mit einem geeigneten Extraktionsmittel vermischt und anschließend die klare, mit aromatischem Carbonat und aromatischer Hydroxyverbindung angereicherte Aufnehmerphase von der katalysatorhaltigen Abgeberphase abtrennt.

Es ist bekannt, organische Carbonate, z.B. Diphenylcarbonat (DPC), durch oxidative Umsetzung einer aromatischen Hydroxyverbindung mit Kohlenmonoxid in Gegenwart eines Edelmetall-Katalysators herzustellen (DE-OS 27 38 437). Als Edelmetall wird bevorzugt Palladium eingesetzt. Zusätzlich können ein Cokatalysator (z.B. Mangan- oder Kobaltsalze), eine Base, ein quaternäres Salz, verschiedene Chinone bzw. Hydrochinone und Trockenmittel eingesetzt werden. Dabei kann in einem Lösungsmittel, bevorzugt Methylenchlorid, gearbeitet werden. Bei der beschriebenen Umsetzung wird nur eine geringe Menge an organischem Carbonat gebildet. Eine Methode zur Abtrennung des Carbonats von der komplexen Reaktionslösung wird nicht offenbart.

In JP-04 257 546 wird ein Verfahren beschrieben, bei dem organische Carbonate durch kontinuierliches Einspeisen der Reaktionspartner in eine Destillationskolonne bei 150-205°C und 30-50 bar hergestellt werden. Das Reaktionswasser wird kontinuierlich abdestilliert. Nachteilig bei diesem Verfahren ist, daß man zur Entfernung des Reaktionswassers in einer Destillationskolonne arbeiten muß, die aufgrund ihrer Konstruktion nur kurze Verweilzeiten ermöglicht. Die mit diesem Verfahren realisierbaren Raum-Zeit-Ausbeuten sind demzufolge mit nur 17,8 g/l h sehr niedrig. Mit der Reaktionsführung in einer Destillationskolonne ist die Verwendung großer Mengen Halogenide bei hohen Temperaturen (150 - 205°C) verbunden. Dies führt zu großen Korrosionsproblemen, die zusätzlich einen hohen apparativen Aufwand bedingen. Dem Fachmann ist außerdem bekannt, daß unter den angegebenen Reaktionsbedingungen bevorzugt als Cokatalysatoren eingesetzte Jodide nicht stabil sind und in erheblichem Maße zu Jod oxidiert werden. Dies führt zu großen Verlusten des Cokatalysators und zur Bildung von Nebenprodukten, was die Selektivität und damit die Wirtschaftlichkeit dieses Verfahrens stark beeinträchtigt. Bei diesen hohen Temperaturen und Drücken findet außerdem eine rasche Desaktivierung des homogenen Katalysatorsystems statt, so daß eine wirtschaftliche Nutzung dieses Verfahrens nicht möglich ist. Weiterhin gelingt hierbei zwar die Abtrennung des Reaktionswassers, für die Isolierung des Diarylcarbonats stellt dies jedoch keine Lösung dar.

In EP 450 442 und EP 507 546 wird die DPC-Isolierung durch Destillation der Reaktionslösung beschrieben. Unter den angegebenen Destillationsbedingungen (ca. 15 Torr und 100-200°C) soll nach mehrstündiger Destillation zunächst Phenol und anschließend schließlich farbloses DPC erhalten werden. In EP 507 546 wird der erhaltene Destillationsrückstand an Luft für 16 h bei 700°C behandelt und soll danach nur noch aus Palladium und Kobalt bestehen. Die hier vorgestellte Vorgehensweise führt zwangsläufig zum vollständigen Verlust der verwendeten Katalysatorkomponenten Tetrabutylammoniumbromid, Benzochinon und Co-acetylacetonat. Neben dem Verlust der Katalysatorkomponenten und den hierdurch ablaufenden Nebenreaktionen läßt sich eine DPC-Ausbeute von lediglich 6-9% errechnen, d.h. nur ein geringer DPC-Anteil ist auf diesem Wege überhaupt isolierbar. Alle diese Nachteile sprechen gegen eine technische Realisierbarkeit, da sie das Aufarbeitungsverfahren teuer und zudem noch unselektiv gestalten.

In EP 583 936 und EP 583 938 wird zur Abtrennung des aromatischen Carbonats die Kristallisation des 1:1-Addukts aus Phenol und Diphenylcarbonat aus dem Reaktionsgemisch vorgeschlagen. Nachteile dieser Verfahren sind die zur Kristallisation notwendige starke Abkühlung, die langsame Kristallisation, die Abhängigkeit von einem hohen DPC-Gehalt in der Reaktionslösung, d.h. lange Reaktionszeiten und der Einschluß von Katalysatorkomponenten in die erhaltenen Kristallisate, d.h. die Trennung ist unvollständig. Bedingt durch die langen Kristallisationszeiten sind für die Aufarbeitung große Behältervolumina notwendig. Der Prozeß wird hierdurch langwierig und kostenintensiv. Diese Nachteile machen bei einer technischen Realisierung das Verfahren sehr teuer und damit ökonomisch nicht sinnvoll.

Es bestand daher die Aufgabe, eine schonende Aufarbeitungsmethode zu finden, die es erlaubt, die Abtrennung von Diarylcarbonaten vom Katalysatorsystem und von der restlichen Reaktionslösung mit hoher Raum-Zeit-Ausbeute und unter wirtschaftlichen, technisch realisierbaren und reproduzierbaren Bedingungen durchzuführen.

Es wurde nun überraschend gefunden, daß die beschriebenen Nachteile überwunden werden können, wenn man die Reaktionslösung aus dem Reaktor entnimmt, mit einem selektiven Extraktionsmittel intensiv vermischt und anschließend die Phasen in eine untere Abgeberphase und eine obere Aufnehmerphase trennt. Die resultierende Abgeberphase ist an aromatischem Carbonat und aromatischer Hydroxyverbindung abgereichert und enthält neben dem Katalysatorsystem ggf. noch einige Prozente gelösten Aufnehmer. Völlig überraschend bestehen die erhaltenen, klaren Aufnehmerphasen neben dem Extraktionsmittel nur aus aromatischer Hydroxyverbindung, aromatischem Carbonat sowie geringen Spuren des Katalysatorsystems. Weiterhin überraschend war die Tatsache, daß sich beim Übergang in die Aufnehmerphase das aromatische Carbonat gegenüber der aromatischen Hydroxyverbindung stark anreichert.

Die Erfindung betrifft demnach ein Verfahren zur gemeinsamen Abtrennung eines Diarylcarbonats der Formel

R¹-O-CO-O-R¹ (I)

und der zugrundeliegenden aromatischen Hydroxyverbindung der Formel

R¹-OH (II),

wobei
- R¹: C₆-C₁₂-Aryl, HO-C₆H₄-C₁-C₆-alkyliden-C₆H₄- oder pro Arylkern 1- bis 2fach durch C₁-C₄-Alkyl, C₁-C₄-Alkoxy oder Halogen substituiertes C₆-C₁₂-Aryl oder derart substituiertes HO-C₆H₄-C₁-C₆-alkyliden-C₆H₄- bedeutet,
aus rohen Reaktionsgemischen der oxidativen Umsetzung der aromatischen Hydroxyverbindung (II) mit Kohlenmonoxid in Gegenwart eines Edelmetall enthaltenden Katalysatorsystems, das dadurch gekennzeichnet ist, daß das rohe Reaktionsgemisch bei 20-150°C, bevorzugt 30-120°C, besonders bevorzugt 40-100°C und 1-200 bar, bevorzugt 1-100 bar, besonders bevorzugt 1-50 bar mit einer Gewichtsmenge von 0,1-20 Teilen, bevorzugt 0,2 bis 10 Teile, besonders bevorzugt 0,3 bis 5 Teile, bezogen auf 1 Teil Reaktionsgemisch, an einem unpolaren, aprotischen Extraktionsmittel vermischt wird, anschließend bei 20-150°C eine sich spontan einstellende Phasentrennung in eine untere Abgeberphase und eine obere Aufnehmerphase vorgenommen wird und das Diarylcarbonat (I) und die aromatische Hydroxyverbindung aus der Aufnehmerphase isoliert werden.

Sowohl das Vermischen als auch die Phasentrennung im erfindungsgemäßen Verfahren hängen selbstverständlich von der Größe und der Zusammensetzung des Reaktionsansatzes ab. Die Untergrenze beträgt 0,05 h, die Obergrenze überschreitet 30 h nicht, bevorzugt 10 h nicht.

Das erfindungsgemäß zu behandelnde rohe Reaktionsgemisch enthält 0,1-3 Gew.-%, bevorzugt 1-2 Gew.-% quaternäres Salz, 0,1-2 Gew.-%, bevorzugt 0,5-1,5 Gew.-% Wasser, 5-500 ppm, bevorzugt 20-250 ppm Pd, 10-1000 ppm, bevorzugt 250-750 ppm Cokatalysator, 0,01-1 Gew.-%, bevorzugt 0,1-0,5 Gew.-% Base und 95-98,5 Gew.-% zusammen an aromatischer Hydroxyverbindung und Diarylcarbonat.

Die Phasentrennung stellt sich spontan ein, kann aber auch etwa durch Zentrifugieren unterstützt werden.

Die so erhaltenen beladenen Aufnehmerlösungen lassen sich problemlos weiterverarbeiten, beispielsweise durch Destillation oder/und Kristallisationen, da sie keine störenden Katalysatorkonzentrationen mehr enthalten.

Die abgetrennte Diarylcarbonatmenge kann hierbei 2 bis 100%, bevorzugt 5 bis 90, besonders bevorzugt 5 bis 80 % des im Rohprodukt enthaltenen Diarylcarbonats betragen. Das Verhältnis von Diarylcarbonat zu aromatischer Hydroxyverbindung im beladenen Aufnehmer liegt zwischen 0,001 bis 30, bevorzugt 0,01 bis 15.

Das erfindungsgemäße Verfahren läßt sich zur partiellen oder vollständigen Abtrennung von Diarylcarbonat-Rohprodukt aus der oxidativen Carbonylierung von aromatischen Hydroxyverbindungen, beispielsweise von Phenol zu Diphenylcarbonat, anwenden. Der Diarylcarbonatgehalt der Rohlösung kann je nach Reaktionsbedingungen, Laufzeit und Katalysatorkonzentration bei 5 bis 95 Gew. % und die Konzentration an aromatischer Hydroxyverbindung zwischen 95 und 5 Gew.% liegen, bezogen auf die Summe an Diarylcarbonat und aromatischer Hydroxyverbindung.

Eine verdünnte Reaktionslösung kann auch mit Hilfe einer schonenden Destillation auf den gewünschten Diarylcarbonatgehalt aufkonzentriert werden. In einer weiteren Ausführungsform läßt sich der gewünschte DPC-Gehalt vor der Extraktion durch DPC-Zusatz zur Reaktionslösung einstellen.

Die während des gesamten Extraktionsvorgangs überlagerte Gasphase kann stationär sein oder ständig erneuert werden und aus dem Reaktionsgas, einem Gemisch aus Reaktionsgas und Inertgas oder einem Inertgas bestehen. Als Inertgas im erfindungsgemäßen Verfahren sind Stickstoff, Wasserstoff, Kohlendioxid, Edelgase sowie unter den Extraktionsbedingungen beständige und nicht störende organische Verbindungen einsetzbar. In einfacher und damit gleichzeitig vorteilhafter Weise wird das erfindungsgemäße Verfahren unter CO oder dem Reaktionsgas CO/O₂ bzw. CO/O₂/Inertgas durchgeführt.

Die erfindungsgemäße Extraktion läßt sich auf Reaktionslösungen der oxidativen Carbonylierung von aromatischen Hydroxyverbindungen anwenden, wobei als aromatische Hydroxyverbindungen beispielsweise Phenol, o-, m- oder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol, 2-Naphthol und Bisphenol A, bevorzugt Phenol genannt seien und das entsprechende Diarylcarbonat enthalten ist. Allgemein handelt es sich im Falle einer Substitution der aromatischen Hydroxyverbindung um 1 oder 2 Substituenten in der Bedeutung von C₁-C₄-Alkyl, C₁-C₄-Alkoxy oder Halogen, wie Fluor, Chlor oder Brom. C₆-C₁₂-Aryl ist allgemein Phenyl, Naphthyl oder Biphenyl. Die Gruppe HO-C₆H₄-C₁-C₆-alkyliden-C₆H₄- ist der Rest eines Bisphenols, das durch Kondensation von 2 Molekülen eines gegebenenfalls im angegebenen Umfang substituierten Phenols mit einer Oxoverbindung erhältlich ist. Oxoverbindungen mit 1-6 C-Atomen hierfür sind beispielsweise Formaldehyd, Acetaldehyd und weitere aliphatische Aldehyde bis zum Capronaldehyd sowie Aceton, Methyl-ethyl-Keton, Diethylketon und aliphatische C₅- oder C₆-Ketone mit homologem Aufbau sowie Cyclopentanon und Cyclohexanon.

Bevorzugte Diarylcarbonate und zugrundeliegende aromatische Hydroxyverbindungen sind allgemein solche der Formeln

R²-O-CO-O-R² (III) bzw. R²-OH (IV),

in denen
- R²: Phenyl oder 1- bis 2fach durch C₁-C₄-Alkyl, C₁-C₄-Alkoxy oder Halogen substituiertes Phenyl, bevorzugt nicht substituiertes Phenyl, darstellt.

Als Extraktionsmittel eignen sich unpolare und aprotische Verbindungen, wie beispielsweise halogenierte (fluorierte oder chlorierte) Kohlenwasserstoffe mit 3-100 Kohlenstoffatomen, verzweigte und gradkettige Alkane mit 3-100 Kohlenstoffatomen, wie z.B. Propan, Butane, Pentane, Hexane, Heptane, Octane, wie beispielsweise n-Octan und 2,2,4-Trimethylpentan, Nonane, Decane, Undecane, Dodecane, Tridecan, Tetradecan, Pentadecane, Hexadecane, Heptadecane, Octadecane, Nonadecane, Eicosane, Triacontane, Tetracontane und Pentacontane, verzweigte und unverzeigte cycloaliphatische Verbindungen mit 3-100 Kohlenstoffatomen, wie Cyclobutan, Cyclopentan, Cyclohexan, Cycloheptan, Cyclooctan, Cyclononan, Cycloundecan, Cyclododecan, Cyclotridecan, Methylcyclopentan, Methylcyclohexan, Dimethylcyclopentane, Dimethylcyclohexane, Tetralin, Dekalin als Reinsubstanzen, Gemische dieser Reinsubstanzen oder Fraktionen der Rohöldestillation, wie beispielsweise Petroletherfraktionen, Ligroin, Rohbenzin, Gasöl oder Dieselöl, bevorzugt werden Hexan-, Heptan-, Octan-, Nonan-, Decan- und Undecanfraktionen, Petroletherfraktionen, Ligroin und Rohbenzin, Gasöl, Dieselöl, 2,2,4-Trimethylpentan und Dodecane, besonders bevorzugt 2,2,4-Trimethylpentan und Dodecane. In bevorzugter Weise haben die Halogenkohlenwasserstoffe, Alkane und cycloaliphatischen Verbindungen 3-30, besonders bevorzugt 3-20 Kohlenstoffatome. Alle diese Stoffe bilden mit dem rohen Reaktionsgemisch ein Zwei-Phasen-System aus Abgeberphase und Aufnehmerphase.

Für das erfindungsgemäße Verfahren zur extraktiven Abtrennung von Diarylcarbonaten und aromatischen Hydroxyverbindungen aus den Rohlösungen können Extraktionsverfahren eingesetzt werden, wie sie beispielsweise in KIRK-OTHMER, Encyclopedia of Chemical Technology, Fourth Edition, Volume 10, 1993, Seiten 125-181 und in Ullmann's Encyclopedia of Industrial Chemistry, Fifth Edition, Volume B3, Unit Operations II, 1988, Kapitel 6, Liquid-Liquid-Extraktion, Seiten 6-1 - 6-61 beschrieben sind. So können beispielsweise Extraktionsapparate der folgenden Klassifizierungsgruppen zur Anwendung kommen: Kolonnen ohne Energieeintrag, Kolonnen mit gepulster Flüssigkeit oder gepulsten Einbauten, Kolonnen mit rotierenden Einbauten, Mixer-Settler sowie Zentrifugalextraktoren. Als Beispiele für Kolonnen ohne Energieeintrag seien Sprühkolonnen, gepackte Kolonnen und Siebbodenkolonnen genannt, die sich in der Dispergierung der Phasen unterscheiden. Als Beispiele für Kolonnen mit gepulster Flüssigkeit oder gepulsten Einbauten seien gepulste Siebbodenkolonnen, mit Kolbenpumpe, mit Pulsator nach Misek oder Wepuko, Kolonnen mit schwingenden Siebböden nach Prochazka bzw. Karr genannt. Als Beispiele für Kolonnen mit rotierenden Einbauten seinen Rotating Disc Contactor (RDC), Asymmetric Rotating Disc Extraktor (ARD), Oldshue-Rushton multiple-mixer Kolonne, Kuhni Extraktor, Scheibel Kolonne, SHE Extraktor und Graesser Contactor genannt. Als Beispiele für Mixer-Settler-Extraktoren seien Davy McKee-Mixer-Settler, Lurgi-Turmextraktor, IMI, General Mills und Boxtyp-Mixer-Settler nach Denver genannt. Als Beispiele für Zentrifugalextraktoren seien Podbielniak-Zentrifugalextraktor und Robatel-Zentrifugalextraktor genannt. Die Extraktoren können hierbei als Einzelextraktoren, extraktore, parallele Extraktoren oder als Kaskaden von Extraktoren betrieben werden. Bei der Anwendung von Kaskaden von Extraktionsapparaten können Apparate einer oder verschiedener Klassifizierungsgruppen gleichzeitig in einer Kaskade betrieben werden. Die Führung der Abgeber- und Aufnehmerphase kann in einer Kaskade im Gleichstrom oder bevorzugt im Gegenstrom erfolgen.

Das erfindungsgemäße Extraktionsverfahren läßt sich diskontinuierlich und kontinuierlich, bevorzugt kontinuierlich durchführen.

Im erfindungsgemäßen Verfahren verbleibt das Katalysatorsystem, bestehend aus Edelmetallverbindung-Katalysator, Cokatalysator, quaternärem Salz und Base, in der Abgeberphase. Dieses Katalysatorsystem ist nach wie vor aktiv und kann daher in die Herstellung von Diarylcarbonat recyclisiert werden. Geringe Aktivitätseinbußen können durch Teilausschleusung (Purge) und entsprechenden Ersatz durch frisches Katalysatorsystem ausgeglichen werden. Ansonsten wird die gesamte Abgeberphase wieder der Herstellung von Diarylcarbonat zugeführt. Das erfindungsgemäße Verfahren stellt somit gleichzeitig eine schonende Abtrennung des gesamten Katalysatorsystems vom hergestellten Diarylcarbonat und überschüssiger aromatischer Hydroxyverbindung dar.

Die folgenden Beispiele verdeutlichen das erfindungsgemäße Verfahren, ohne es jedoch auf diese einzuschränken.

### Beispiele

Im folgenden werden als Abkürzungen benutzt:
DPC = Diphenylcarbonat, Ph = Phenyl, TBAB = Tetrabutylammoniumbromid
AAS = Atomabsorptionsspektrum

### Beispiel 1- 9:

In eine Apparatur, bestehend aus einem beheizten 200 ml-Planschliffgefäß mit Rührer, Wellenbrecher, Kühler mit nachgeschalteter Kühlfalle und Abgasanschluß, Temperaturmessung, Begasungseinheit und Einfüllstutzen, wurden nacheinander 50 g Reaktionslösung und 50 g bzw. 25 g Extraktionsmittel eingefüllt. Die Reaktionslösung hatte folgende Zusammensetzung: 15 % DPC, 81,8 % PhOH, 1,0 % H₂O und 2,2 % Katalysatorsystem (TBAB, NaOPh, Mn- und Pd-Verbindung). Das DPC:PhOH-Verhältnis betrug 0,18. Die auf 50°C temperierte Lösung wurde 60 Minuten intensiv durchmischt und dann der Rührer abgeschaltet. Die Phasentrennung setzte sofort ein. Die untere, schwarz gefärbte Abgeberphase enthielt das Katalysatorsystem, die obere klare Lösung bildete die Aufnehmerphase. Die Lösungen wurden getrennt voneinander mit Hilfe der Gaschromatographie untersucht. Die Ergebnisse sind in Tabelle 1 zusammengefaßt.

### Beispiel 10 - 13:

In die Apparatur aus Beispiel 1 wurden nacheinander 50 g Reaktionslösung und 50 g 2,2,4-Trimethylpentan eingefüllt. Die Reaktionslösung hatte folgende Zusammensetzung: 15 % DPC, 81,8 % PhOH, 1,0 % H₂O und 2,2 % Katalysatorsystem (TBAB, NaOPh, Mn- und Pd-Verbindung). Das DPC:Phenol-Verhältnis betrug 0,18. Die auf 70°C temperierte Lösung wurde intensiv durchmischt, auf die gewünschte Temperatur abgekühlt und der Rührer abgeschaltet. Die Phasentrennung setzte sofort ein. Die untere, schwarz gefärbte Abgeberphase enthielt das Katalysatorsystem, die obere, klare Lösung bildete die Aufnehmerphase. Die Lösungen wurden getrennt voneinander mit Hilfe der Gaschromatographie untersucht. Die Ergebnisse sind in Tabelle 2 zusammengefaßt.

### Beispiel 14 - 16:

In der Apparatur aus Beispiel 1 wurden nacheinander 50 g Reaktionslösung und 50 g eines Dodecan-Isomerengemischs eingefüllt. Die Reaktionslösungen besaßen die aus Tabelle 3 ersichtlichen Zusammensetzungen. Die auf 70°C temperierte Lösung wurde intensiv durchmischt, auf die gewünschte Temperatur abgekühlt und der Rührer abgeschaltet. Die Phasentrennung setzte sofort ein. Die untere, schwarz gefärbte Abgeberphase enthielt das Katalysatorsystem, die obere, klare Lösung bildete die Aufnehmerphase. Die Lösungen wurden getrennt voneinander mit Hilfe der Gaschromatographie untersucht. Die Ergebnisse sind in Tabelle 3 zusammengefaßt.

### Beispiel 17:

In eine Apparatur, bestehend aus einem beheizten 3 1-Planschliffgefäß mit Rührer, Bodenablaßventil, Kühler mit nachgeschalteter Kühlfalle und Abgasanschluß, Temperaturmessung, Begasungseinheit und Einfüllstutzen, an dessen Bodenventil ein beheizter 3 1-Scheidetrichter mit Dreiwegehahn und zwei 2 1-Rundkolben mit Abgasanschluß angeschlossen waren, wurden nacheinander 1000 g Reaktionslösung und 1000 g 2,2,4-Trimethylpentan eingefüllt. Die Reaktionslösung hatte folgende Zusammensetzung: 41,7 % DPC, 55,1 % PhOH, 1,0 % H₂O und 2,2 % Katalysatorsystem (TBAB, NaOPh, Mn- und Pd-Verbindung). Das DPC:Phenol-Verhältnis betrug 0,76. Die auf 70°C temperierte Lösung wurde 10 Minuten intensiv durchmischt, auf 50°C abgekühlt und rasch in den Scheidetrichter (50°C) übergeführt. Die Phasentrennung setzte sofort ein. Die untere, schwarz gefärbte Abgeberphase enthielt das Katalysatorsystem, die obere, klare Lösung bildete die Aufnehmerphase. Die Lösungen wurden getrennt voneinander je in einem 2 1-Kolben aufgefangen. Nach der Extraktion betrug das Gewicht der Abgeberphase 984,3 g, das der Aufnehmerphase 1015,7 g. Die Lösungen wurden destillativ vom Extraktionsmittel befreit und anschließend gaschromatographisch analysiert. In der Aufnehmerphase befanden sich demnach 112,1 g Phenol und 103,6 g DPC (DPC:PhOH-Verhältnis = 0,92), d.h. DPC wurde im Aufnehmer angereichert. Das so erhaltene weiße Extrakt enthielt nur noch Spuren an TBAB. AAS-Untersuchungen ergaben nur Spuren an Mn, kein Pd und nur geringe Mengen Na. Durch diese einmalige Extraktion wurden bereits ca. 25% des DPC entnommen. In der Abgeberphase waren ca. 200 g Extraktionsmittel gelöst.

### Beispiel 18:

Analog zu Beispiel 17 wurden 1000 g eines Reaktionsprodukts mit 66,5 % DPC (DPC:PhOH-Verhältnis betrug ca. 2,1) aufgearbeitet. Die Lösungen wurden destillativ vom Extraktionsmittel befreit und anschließend gaschromatographisch analysiert. In der Aufnehmerphase befanden sich demnach 101,7 g Phenol und 256,8 g DPC (DPC:PhOH-Verhältnis = 2,53), d.h. DPC wurde im Aufnehmer angereichert. Das so erhaltene weiße Extrakt enthielt nur noch Spuren an TBAB. AAS-Untersuchungen ergaben nur Spuren an Mn, kein Pd und nur geringe Mengen Na. Durch diese einmalige Extraktion wurden ca. 38,6 % des DPC entnommen. In der Abgeberphase waren ca. 195 g Extraktionsmittel gelöst.

### Beispiel 19:

Analog zu Beispiel 17 wurden 897,8 g eines Reaktionsprodukts mit 9,7 % DPC (DPC:PhOH-Verhältnis = ca. 0,1) mit 897,8 g Isooctan aufgearbeitet. Nach der Extraktion betrug das Gewicht der Abgeberphase 870,8 g, das der Aufnehmerphase 914,4 g. Die Lösungen wurden destillativ vom Extraktionsmittel befreit und anschließend gaschromatographisch analysiert. In der Aufnehmerphase befanden sich demnach 107,1 g Phenol und 24,8 g DPC (DPC:PhOH-Verhältnis war 0,23), d.h. DPC wurde im Aufnehmer angereichert. Das so erhaltene weiße Extrakt enthielt nur noch Spuren an TBAB. AAS-Untersuchungen ergaben nur Spuren an Mn, kein Pd und nur geringe Mengen Na. Durch diese einmalige Extraktion wurden ca. 28,3 % des DPC entnommen. In der Abgeberphase waren ca. 114 g Extraktionsmittel gelöst.

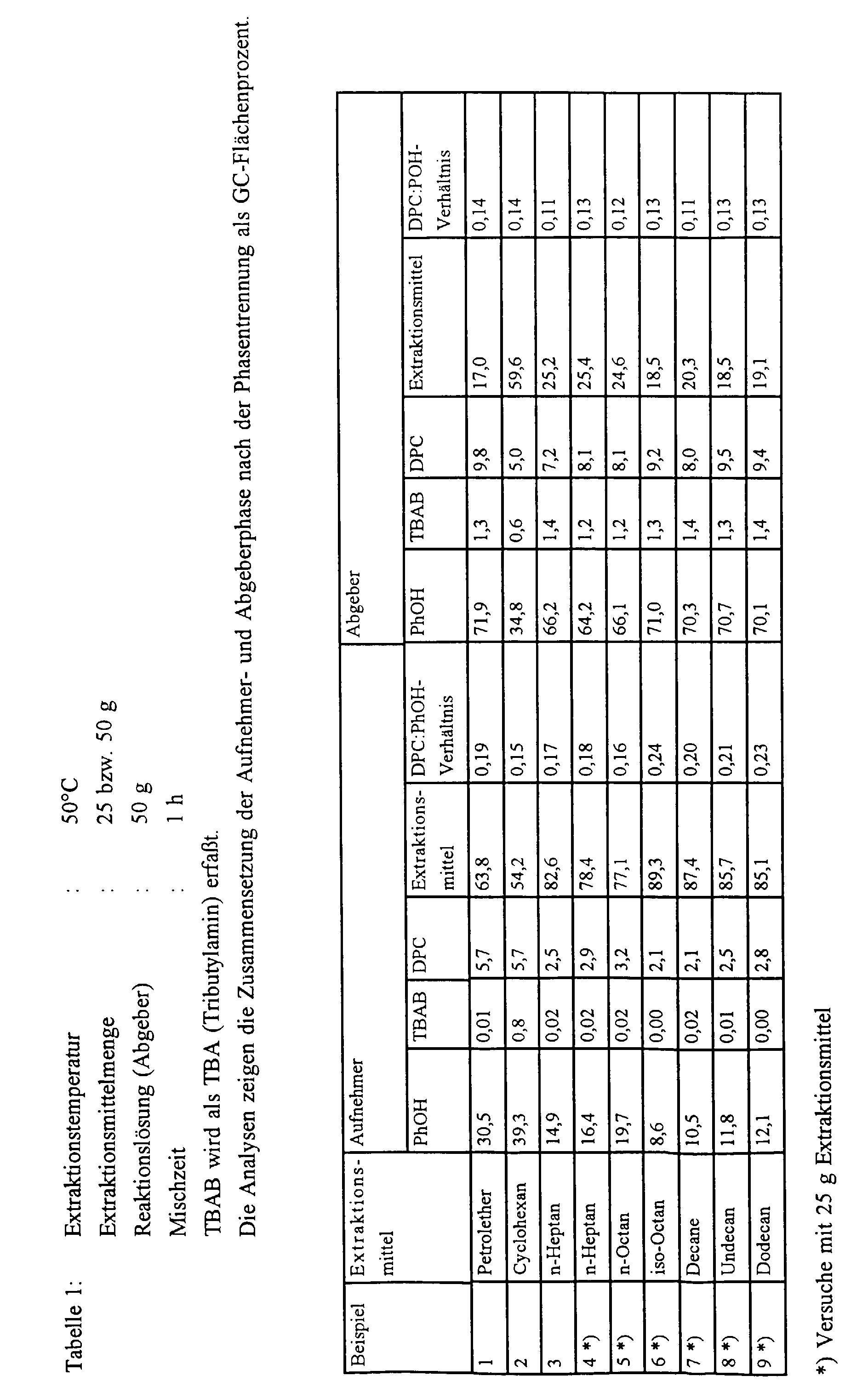

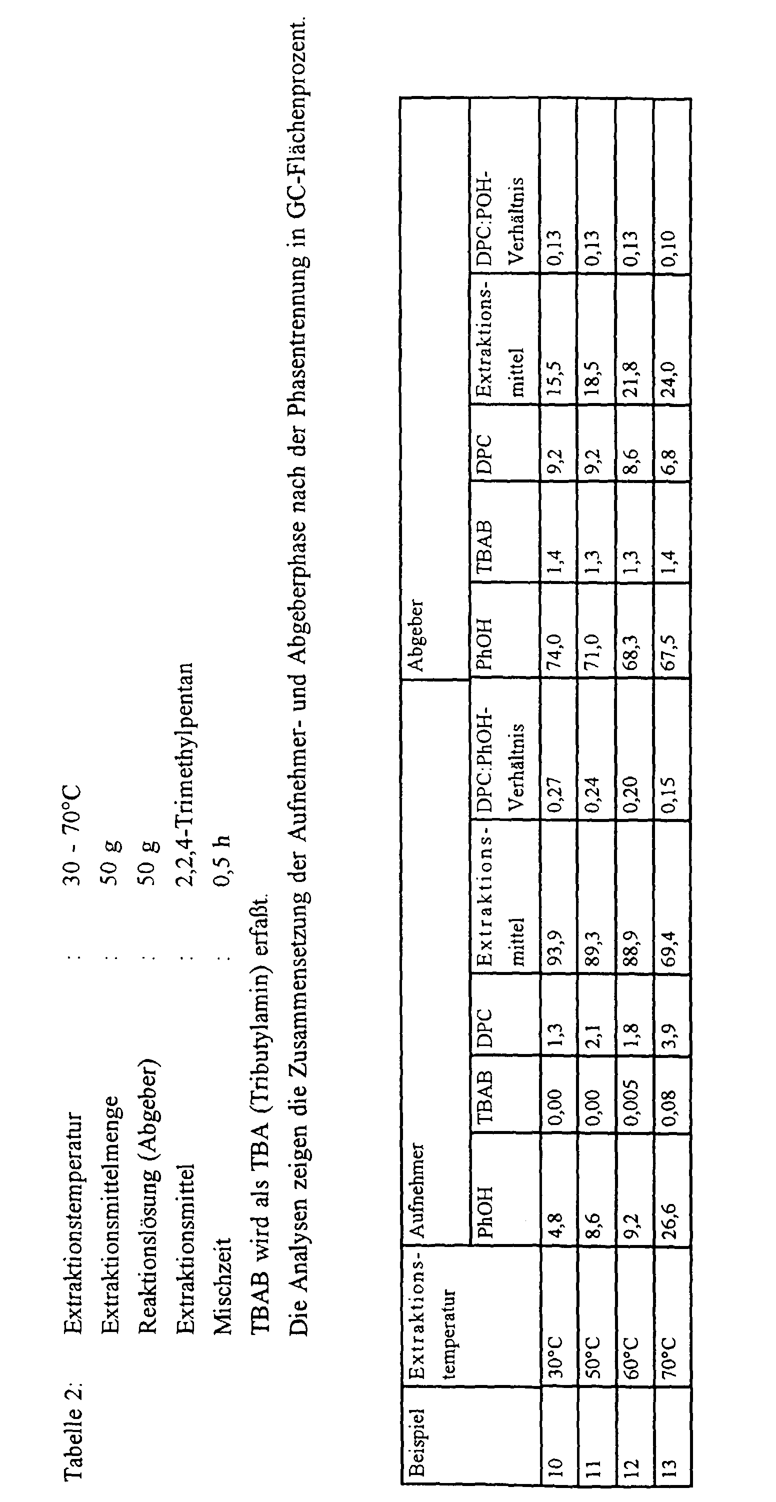

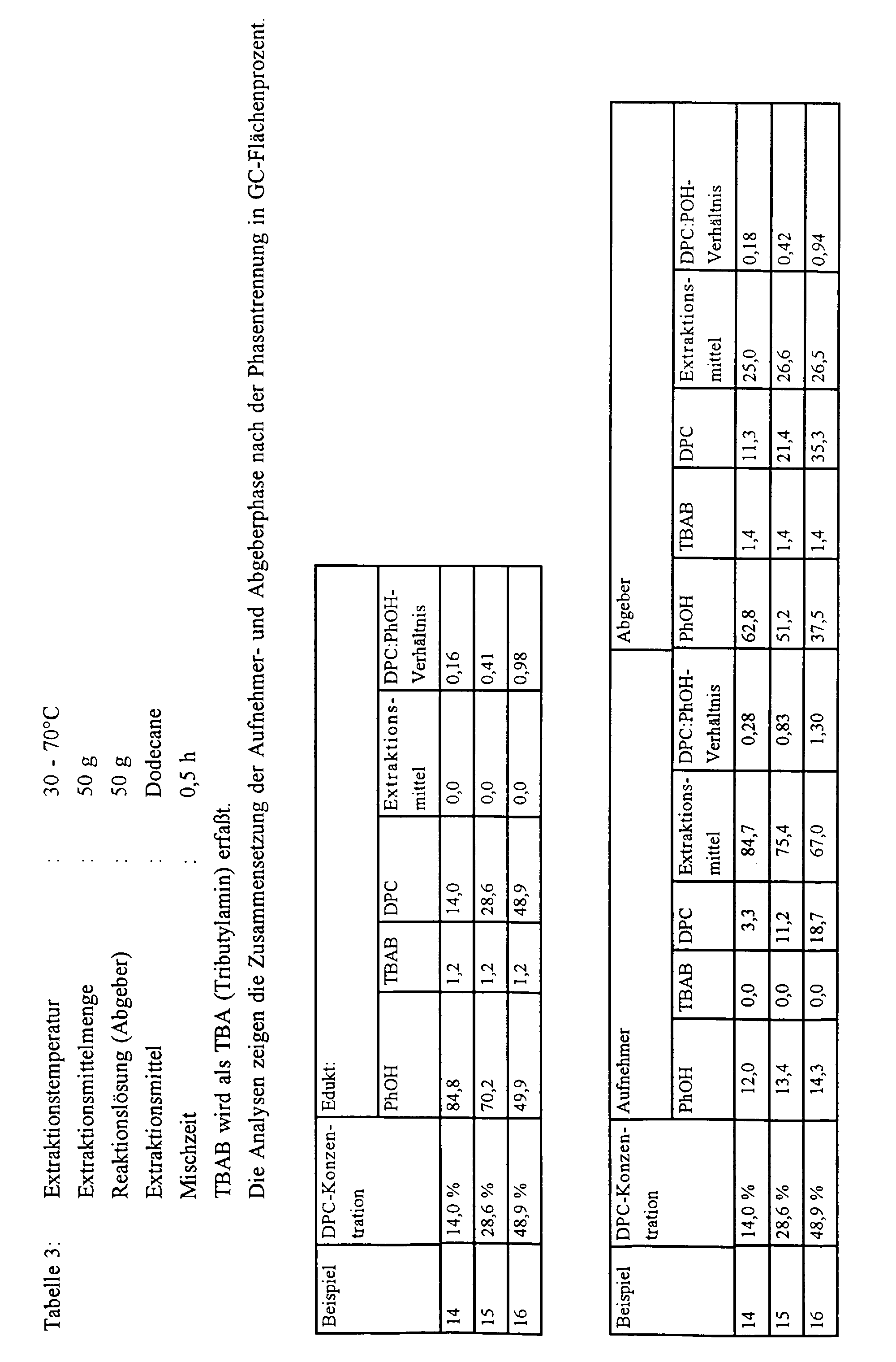

## Patentansprüche

1. Verfahren zur gemeinsamen Abtrennung eines Diarylcarbonats der Formel
R¹-O-CO-O-R¹ (I)
und der zugrundeliegenden aromatischen Hydroxyverbindung der Formel
R¹-OH (II),
wobei
R¹ C₆-C₁₂-Aryl, HO-C₆H₄-C₁-C₆-alkyliden-C₆H₄- oder pro Arylkern 1-bis 2fach durch C₁-C₄-Alkyl, C₁-C₄-Alkoxy oder Halogen substituiertes C₆-C₁₂-Aryl oder derart substituiertes HO-C₆H₄-C₁-C₆-alkyliden-C₆H₄- bedeutet,
aus rohen Reaktionsgemischen der oxidativen Umsetzung der aromatischen Hydroxyverbindung (II) mit Kohlenmonoxid in Gegenwart eines Edelmetall enthaltenden Katalysatorsystems dadurch gekennzeichnet, daß das rohe Reaktionsgemisch bei 20-150°C und 1-200 bar mit einer Gewichtsmenge von 0,1-20 Teilen, bezogen auf 1 Teil Reaktionsgemisch, an einem unpolaren, aprotischen Extraktionsmittel vermischt wird, anschließend bei 20-150°C eine sich spontan einstellende Phasentrennung in eine untere Abgeberphase und eine obere Aufnehmerphase vorgenommen wird und das Diarylcarbonat (I) und die aromatische Hydroxyverbindung aus der Aufnehmerphase isoliert werden.

2. Verfahren nach Anspruch 1, dadurch gekennzeichnet, daß das Diarylcarbonat und die aromatische Hydroxyverbindung solche der Formeln
R²-O-CO-O-R² (III) bzw. R²-OH (IV),
sind, in denen
R² Phenyl oder 1- bis 2fach durch C₁-C₄-Alkyl, C₁-C₄-Alkoxy oder Halogen substituiertes Phenyl darstellt.

3. Verfahren nach Anspruch 1, dadurch gekennzeichnet, daß als Extraktionsmittel halogenierte Kohlenwasserstoffe, Alkane und cycloaliphatische Verbindungen mit 3-100 Kohlenstoffatomen eingesetzt werden.

## Claims

1. Process for simultaneously separating a diaryl carbonate of the formula
R¹-O-CO-O-R¹ (I)
and the underlying aromatic hydroxy compound of the formula
R¹-OH (II),
wherein
R¹ means C₆-C₁₂ aryl, HO-C₆H₄-C₁-C₆-alkylidene-C₆H₄-or C₆-C₁₂-aryl mono- to di-substituted in each aryl ring by C₁-C₄-alkyl, C₁-C₄-alkoxy or halogen or similarly substituted HO-C₆H₄-C₁-C₆-alkylidene-C₆H₄,
from crude reaction mixtures from the oxidative reaction of the aromatic hydroxy compound (II) with carbon monoxide in the presence of a catalyst system containing noble metal, characterised in that the crude reaction mixture is mixed with a proportion by weight of 0.1-20 parts, relative to 1 part of reaction mixture, with a non-polar, aprotic extracting agent at 20-150°C and 1-200 bar, whereupon spontaneous phase separation into a lower donor phase and an upper recipient phase is brought about at 20-150°C and the diaryl carbonate (I) and the aromatic hydroxy compound are isolated from the recipient phase.

2. Process according to claim 1, characterised in that the diaryl carbonate and the aromatic hydroxy compound are those of the formulae
R²-O-CO-O-R² (III) and R²-OH (IV)
respectively, in which
R² represents phenyl or phenyl mono- to di-substituted by C₁-C₄-alkyl, C₁-C₄-alkoxy or halogen.

3. Process according to claim 1, characterised in that halogenated hydrocarbons, alkanes and cycloaliphatic compounds having 3-100 carbon atoms are used as the extracting agents.

## Revendications

1. Procédé pour séparer ensemble un carbonate de diaryle de formule:
R¹-O-CO-O-R¹ (I)
et le composé aromatique hydroxylé correspondant de formule :
R¹-OH (II)
dans lesquelles
R¹ représente un groupe aryle en C₆-C₁₂, un groupe HO-C₆H₄-alkylidène en C₁-C₆-, C₆H₄- ou un groupe aryle en C₆-C₁₂ portant 1 à 2 substituants alkyles en C₁-C₄, alcoxy en C₁-C₄ ou halogéno par noyau aryle, ou un groupe HO-C₆H₄-alkylidène en C₁-C₆-C₆H₄- portant de tels substituants,
à partir de mélanges de réaction bruts obtenus à la réaction oxydante du composé aromatique hydroxylé (II) avec le monoxyde de carbone en présence d'un système catalyseur contenant un métal noble, caractérisé en ce que l'on ajoute au mélange de réaction brut à des températures de 20 à 150°C et des pressions de 1 à 200 bars, et on mélange, une quantité de 0,1 à 20 parties, pour 1 partie du mélange de réaction, d'un agent d'extraction aprotonique non polaire, on laisse ensuite le mélange se décanter spontanément à des températures de 20 à 150°C en une phase inférieure extraite et une phase supérieure extractrice et on isole le carbonate de diaryle (I) et le composé aromatique hydroxylé de la phase extractrice.

2. Procédé selon la revendication 1, caractérisé en ce que le carbonate de diaryle et le composé aromatique hydroxylé répondent aux formules respectives
R²-O-CO-O-R² (III) et R²-OH (IV),
dans lesquelles
R² représente un groupe phényle ou un groupe phényle portant 1 à 2 substituants alkyles en C₁-C₄, alcoxy en C₁-C₄ ou halogéno.

3. Procédé selon la revendication 1, caractérisé en ce que l'agent d'extraction utilisé consiste en un hydrocarbure halogéné, un alcane ou un dérivé cycloaliphatique contenant 3 à 100 atomes de carbone.
